Europäisches Patentamt

European Patent Office

Office européen des brevets

(19)

(.·) Veröffentlichungsnummer: **0 304 897**

**A2**

## (12) EUROPÄISCHE PATENTANMELDUNG

(21) Anmeldenummer: 88113801.0

(22) Anmeldetag: 24.08.88

(51) Int. Cl.⁴: **A61K 35/74** , //(**A61K35/74, 31:205,31:375)**

(30) Priorität: 25.08.87 DE 3728367

(43) Veröffentlichungstag der Anmeldung:
01.03.89 Patentblatt 89/09

(84) Benannte Vertragsstaaten:
**AT BE CH DE ES FR GB GR IT LI LU NL SE**

(71) Anmelder: **Steglich, Burkhard**
**Kottwitzstrasse 45**
**D-2000 Hamburg 20(DE)**

(72) Erfinder: **Steglich, Burkhard**
**Kottwitzstrasse 45**
**D-2000 Hamburg 20(DE)**

(74) Vertreter: **Harders, Gerhard, Dr. et al**
**Stettiner Strasse 2**
**D-6367 Karben 6(DE)**

(54) **Mittel zur Immunisierung des menschlichen Körpers bei HIV-Infektionen.**

(57) Ein Mittel zur Immunisierung des menschlichen Körpers bei HIV-Infektionen besteht aus
$0,1 - 20 \times 10^6$ Keimen von Mycobacterium Phlei F.U 36,
50 - 1000 mg Diisopropylammoniumdichloracetat und
0,1 - 10 g Ascorbinsäure
pro Tagesdosis.

EP 0 304 897 A2

## Mittel zur Immunisierung des menschlichen Körpers bei HIV-Infektionen

Die vorliegende Erfindung betrifft ein Mittel zur Immunisierung des menschlichen Körpers bei HIV-Infektionen sowie die Verwendung des Mittels bei der Bekämpfung von AIDS.

Bei HIV-Infektionen ist es bisher nur unvollkommen gelungen, die Immunisierungskräfte des menschlichen Körpers so anzuregen, daß ein dauerhafter Erfolg erzielt werden kann. Eine andauernde Reaktivierung des Immunsystems ist bei solchen Infektionen bisher nicht beobachtet worden.

Aufgabe der vorliegenden Erfindung ist die Schaffung eines Mittels, mit dem mit dem bei HIV-Infektionen eine andauernde Reaktivierung des Immunsystems erzielt werden kann.

Gelöst wird diese Aufgabe durch ein Mittel, das aus

0,1 - 20 x $10^6$ Keimen von Mycobacterium Phlei F.U. 36,

50 - 1000 mg Diisopropylammoniumdichloracetat und

1 - 10 g Ascorbinsäure

pro Tagesdosis besteht.

Bevorzugt wird folgende Tagesdosis verwendet:

0,5 - 10 x $10^6$ Keime von Mycobacterium Phlei F.U. 36,

100 - 600 mg Diisopropylammoniumdichloracetat und

1 - 7 g Ascorbinsäure.

Besonders bevorzugt ist ein Mittel mit der folgenden Tagesdosis:

1 - 10 x $10^6$ Keime von Mycobacterium Phlei F.U. 36,

150 - 400 mg Diisopropylammoniumdichloracetat und

1 - 5 g Ascorbinsäure.

Das erfindungsgemäße Mittel eignet sich zur Behandlung von HIV-Infektionen.

Es hat sich überraschenderweise gezeigt, daß die bekannte immunisierende Wirkung des nicht pathogenen Bacterium Phlei durch die Kombination mit Diisopropylammoniumdichloracetat und Ascorbinsäure wesentlich verstärkt wird. Über eine solche Verstärkungswirkung des Diisopropylammoniumdichloracetats und der Ascorbinsäure war bisher nichts bekannt.

Die erfindungsgemäße Kombination kann in allen üblichen Anwendungsformen eingesetzt werden, beispielsweise in Form von wässrigen Lösungen bzw. Suspensionen oder mit Trägerstoffen in Form von Injektionslösungen, Kapseln, Tabletten und dergl.

Es ist möglich, die Tagesdosis auf mehrere Einzeldosen zu verteilen.

Eine Dosierungsform, die sich besonders zur andauernden Reaktivierung des Immunsystems bei HIV-Infektionen eignet, enthält etwa 10 x $10^6$ Keime von Mycobacterium Phlei F.U. 36 sowie 400 mg Diisopropylammoniumdichloracetat und 3 g Ascorbinsäure. Eine weitere Dosierungsform, die insbesondere für die längere Behandlung von HIV-Infektionen geeignet ist, enthält 5 x $10^6$ Keime von Mycobacterium Phlei F.U. 36 sowie 100 bis 200 mg Diisopropylammoniumdichloracetat und 3 g Ascorbinsäure. Dabei werden die niedrigeren Dosen des Diisopropylammoniumdichloracetats für eine längere Anwendung bevorzugt.

Die Behandlung kann auch mit der Gabe von häufigeren Einzeldosen von Diisopropylammoniumdichloracetat und Ascorbinsäure ohne die Keime des Mycobacterium Phlei kombiniert werden, jedoch sollte mindestens etwa an jedem zweiten Tag eine Einzeldosis des erfindungsgemäßen Kombinationspräparats verabreicht werden.

Durch das erfindungsgemäße Kombinationspräparat wird eine optimale Reaktivierung des menschlichen Immunsystems bei HIV-Infektionen erreicht. Schädliche Nebenwirkungen oder Unverträglichkeitsreaktionen wurden bisher nicht beobachtet.

Die Erfindung wird nachfolgend unter Bezugnahme auf die Beispiele, in denen Anwendungsfälle des erfindungsgemäßen Kombinationspräparats bei einzelnen Patienten geschildert sind, näher erläutert. Dabei wird betont, daß alle in den Beispielen enthaltenen Merkmale, insbesondere die verschiedenen Dosierungsangaben, als erfindungswesentlich angesehen werden.

Beispiel 1

Patient A: Der HIV-Test (Elisa) war AK-positiv. Der Wert der OKT4/OKT8-Ratio betrug 1,1.

Der Patient erhielt zwei Monate eine Tagesdosis von 1 x $10^6$ Keimen von Mycobacterium Phlei F.U. 36, 360 mg Diisopropylammoniumdichloracetat und 10 g Ascorbinsäure, verteilt auf zwei Tagesdosen. Am Ende der Behandlung wurde der HIV-Test und die Bestimmung der OKT4/OKT8-Ratio wiederholt. Der HIV-Test war nach wie vor positiv, die OKT4/OKT8-Ratio hatte sich auf 1,8 erhöht.

Die Behandlung wird fortgesetzt.

Beispiel 2

Patient B: Der Patient klagt über Mattheit und Müdigkeit. Es wurde eine vierwöchige Behandlung mit einem Multivitamin-Präparat durchgeführt. Die Behandlung blieb erfolglos, zusätzlich trat Durchfall auf. Ein zu diesem Zeitpunkt durchgeführter HIV-Test war AK-positiv. Der Patient hatte sich offensichtlich während eines 9 Monate vorher stattgefundenen Afrika-Urlaubs infiziert. Die OKT4/OKT8 Ratio wurde mit 1,6 bestimmt.

Der Patient wurde 6 Wochen mit der gleichen Tagesdosis des Kombinationspräparats wie in Beispiel 1 behandelt. Eine erneut durchgeführte Bestimmung der OKT4/OKT8-Ratio ergab einen Wert von 2,7. Auch vorher häufige grippale Infekte sowie chronisch vereiterte Mandeln waren nicht mehr zu beobachten.

Beispiel 3

Patient C: Litt unter allgemeiner Schwäche und Lustlosigkeit. Der HIV-Test (Elisa) war AK-positiv. Der Wert der OKT4/OKT8-Ratio betrug 1,4.

Der Patient wirde 6 Wochen mit dem gleichen Kombinationspräparat wie in Beispiel 1 behandelt. Die erneut durchgeführte Bestimmung der OKT4/OKT8-Ratio ergab 1,8.

Der Patient wurde dann 8 Monate lang mit folgendem Kombinationspräparat weiterbehandelt:
$0,5 \times 10^6$ Keime von Mycobacterium Phlei F.U.
360 mg Diisopropylammoniumdichloracetat
10 g Ascorbinsäure.

Der Patient überstand 8 Monate später eine Lungenentzündung nach dreiwöchiger Behandlung ohne Komplikationen.

**Ansprüche**

1. Mittel zur Immunisierung des menschlichen Körpers bei HIV-Infektionen,
**dadurch gekennzeichnet, daß** es aus
$0,1 - 20 \times 10^6$ Keimen von Mycobacterium Phlei F.U. 36,
50 - 1000 mg Diisopropylammoniumdichloracetat und
0,1 - 10 g Ascorbinsäure
pro Tagesdosis besteht.

2. Mittel nach Anspruch 1, dadurch gekennzeichnet, daß es aus
$0,5 - 10 \times 10^6$ Keimen von Mycobacterium Phlei F.U. 36,
100 - 600 mg Diisopropylammoniumdichloracetat und
1 - 7 g Ascorbinsäure
pro Tagesdosis besteht.

3. Mittel nach Anspruch 1, dadurch gekennzeichnet, daß es aus
$1 - 10 \times 10^6$ Keimen von Mycobacterium Phlei F.U. 36,
150 - 400 mg Diisopropylammoniumdichloracetat und
1 - 5 g Ascorbinsäure
pro Tagesdosis besteht.

4. Verwendung des Mittels nach einem oder mehreren der Ansprüche 1 bis 3 bei der Bekämpfung von AIDS.